(19) Europäisches Patentamt
European Patent Office
Office européen des brevets

(11) Veröffentlichungsnummer : **0 356 703 B1**

(12) # EUROPÄISCHE PATENTSCHRIFT

(45) Veröffentlichungstag der Patentschrift : **02.10.91 Patentblatt 91/40**

(51) Int. Cl.$^5$ : **C07D 307/68**

(21) Anmeldenummer : **89113659.0**

(22) Anmeldetag : **25.07.89**

(54) **Verfahren zur Oxidation von 5-Hydroxymethylfurfural.**

(30) Priorität : **30.07.88 DE 3826073**

(43) Veröffentlichungstag der Anmeldung : **07.03.90 Patentblatt 90/10**

(45) Bekanntmachung des Hinweises auf die Patenterteilung : **02.10.91 Patentblatt 91/40**

(84) Benannte Vertragsstaaten : **AT BE CH DE ES FR GB IT LI NL**

(56) Entgegenhaltungen :
**EP-A- 0 048 974**
**EP-A- 0 073 545**
**GB-A- 2 188 927**
**US-A- 3 326 944**
**US-A- 3 890 381**

(73) Patentinhaber : **HOECHST AKTIENGESELLSCHAFT**
**Postfach 80 03 20**
**W-6230 Frankfurt am Main 80 (DE)**

(72) Erfinder : **Leupold, Ernst Ingo Dr.**
**Am Zäunefeld 15**
**W-6392 Neu-Anspach (DE)**
Erfinder : **Wiesner, Matthias, Dr.**
**Im Münchfeld 8**
**W-6500 Mainz (DE)**
Erfinder : **Schlingmann, Merten, Prof., Dr.**
**Schneidhainer Strasse 32a**
**W-6240 Königstein/Taunus (DE)**
Erfinder : **Rapp, Knut, Dr.**
**Im Kerner 16**
**W-6521 Offstein (DE)**

**EP 0 356 703 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die katalytische Oxidation von 5-Hydroxymethylfurfural zu vielseitig verwendbaren Produkten, die insbesondere als Zwischenprodukte zur Herstellung von Tensiden, Kunststoffen und Harzen eingesetzt werden können.

Es ist bekannt, 5-Hydroxymethylfurfural ohne Mitverwendung von Katalysatoren mit verschiedenen Oxidationsmitteln, wie konzentrierter Salpetersäure (J.J. Blanksma, Chemisches Zentralblatt 1910 I, 539) oder einer Mischung aus Dimethylsulfoxid einerseits und Essigsäureanhydrid Distickstofftetroxid oder Salpetersäure andererseits zu oxidieren (Morikawa, Chem. Abstr. Vol. 92 (1980), 1981 81a).

Bei der Oxidation werden im wesentlichen drei Reaktionsprodukte gefunden :

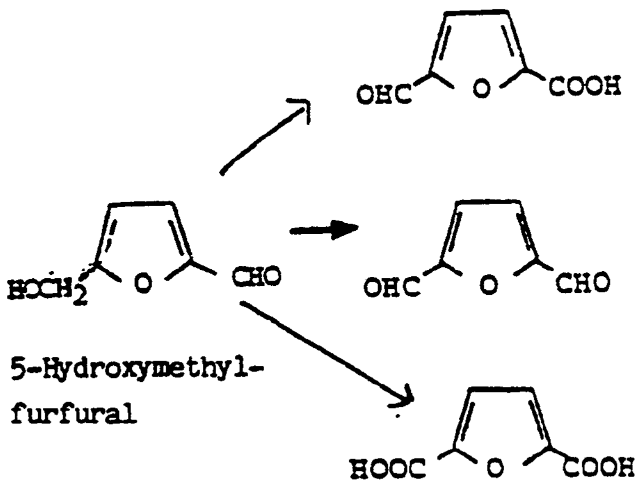

5-Formylfuran-2-carbonsäure

2,5-Diformylfuran

5-Hydroxymethyl-furfural

Furan-2,5-dicarbonsäure

Die genannten Methoden zur Herstellung von Oxidationsprodukten des 5-Hydroxymethylfurfurals sind jedoch mit erheblichen Nachteilen behaftet. Bei Verwendung der herkömmlichen Reagenzien Salpetersäure, Distickstofftetroxid und Dimethylsulfoxid entstehen in Abhängigkeit von den jeweiligen Reagenzien zwangsläufig große Mengen an unerwünschten Produkten, wie nitrose Gase oder Schwefel-Verbindungen, deren Entsorgung einen beträchtlichen Aufwand erfordert. Ebenso ist es sehr aufwendig, bei der Aufarbeitung überschüssiges Oxidationsmittel abzutrennen.

Aus US-A-3326944 ist es ferner bekannt, Hydroxymethylfurfural in stark alkalischem wässrigem Medium (pH-Wert mindestens 12) in Gegenwart eines Platin- oder Palladium-Katalysator mit $O_2$ zu Furandicarbonsäuresalgen zu oxidieren, wobei aber zur Überführung in die freie Säure grosse Mengen Mineralsäure erforderlich sind.

Es wurde nun gefunden dass das betztgenannte Verfahren auch bei einem pH-Wert von höchstens 8 zum Ziele führt.

Gegenstand der Erfindung ist ein Verfahren zur Oxidation mindestens der Hydroxymethylgruppe von 5-Hydroxymethylfurfural, wobei 5-Hydroxymethylfurfural in wäßrigem Medium mit Sauerstoff als Oxidationsmittel in Gegenwart eines Katalysators, der mindestens ein Platinmetall enthält, oxidiert wird, dadurch gekennzeichnet, dass die Oxidation bei einem pH-Wert von höchstens 8 durchgeführt wird.

Als Katalysatoren eignen sich solche, die Metalle der Platingruppe, wie Iridium, Rhodium, Ruthenium, vorteilhaft aber Palladium und/oder Platin, enthalten. Ganz besonders bevorzugt sind Katalysatoren, die als Platinmetall nur Platin enthalten. Vorzugsweise sind die Platinmetalle auf einem Träger, insbesondere auf Aktivkohle aufgebracht. Der Gehalt des Katalysators an dem Metall, insbesondere Platin, liegt vorzugsweise bei 1 bis 10 Gew.-%. Geeignete Katalysatoren sind beispielsweise handelsübliche Katalysatoren mit 5 bis 10 Gew.-% Platin auf Aktivkohle.

Die Konzentration des 5-Hydroxymethylfurfurals in dem wäßrigen Medium kann in weiten Grenzen schwanken. Vorzugsweise wird es in einer Menge von 5 bis 30, insbesondere 10 bis 20 Gew.-%, bezogen auf die Menge von Wasser und Lösungsvermittler, eingesetzt.

Um das Ausfallen von Reaktionsprodukten während der Oxidation zu vermeiden, hat es sich insbesondere bei höheren Konzentrationen als vorteilhaft erwiesen, einen unter den Reaktionsbedingungen gegenüber den Reaktionsteilnehmern inerten Lösungsvermittler, vorzugsweise in einer Konzentration von 10 bis 75 Gew.-%, insbesondere 30 bis 50 Gew.-%, bezogen auf die Menge an Wasser und Lösungsvermittler, einzusetzen. Zweckmäßig werden solche Lösungsvermittler verwendet, die beim Durchleiten von Sauerstoff durch die wäßrige Lösung relativ wenig flüchtig sind, so daß eine Explosionsgefahr im Dampfraum weitgehend vermieden wird ; auf der anderen Seite werden solche Lösungsvermittler bevorzugt, die nach der Oxidation leicht abtrennbar sind, beispielsweise durch Destillation.

Geeignete Lösungsvermittler sind beispielsweise Glykoläther ohne freie OH-Gruppen, wie Glykoläther der Formel $R^1O[CH_2CH(CH_3)O]_nR^2$, wobei n eine ganze Zahl von 1 bis 4 ist und $R^1$ und $R^2$ jeweils unabhängig voneinander $C_1$-$C_4$-Alkyl bedeuten. Besonders geeignet sind die Dimethyl-, Diäthyl- oder Methyl-äthyl-äther und die entsprechenden Propylenglykoläther der genannten allgemeinen Formel mit Siedepunkten im Bereich von 100 bis etwa 250°C, beispielsweise Triäthylenglykoldimethyläther und insbesondere Diäthylenglykoldimethyläther. Auch andere Äther, wie Kronenäther, sind als Lösungsvermittler geeignet, wobei im Einzelfall der Aufwand für die Abtrennung und die Kosten für den Einsatz der Lösungsvermittler bei der Wirtschaftlichkeit des Verfahrens besonders berücksichtigt werden müssen.

Bevorzugtes Oxidationsmittel ist reiner Sauerstoff. Es können jedoch auch Mischungen von Sauerstoff mit unter den Reaktionsbedingungen inerten Gasen, z.B. in Form von Luft, beispielsweise Mischungen von Sauerstoff mit Inertgasen oder mit Luft, verwendet werden.

Im allgemeinen arbeitet man bei einem Gesamtdruck zwischen 0,5 und 100 bar. Bei steigendem Sauerstoffpartialdruck steigt die Reaktionsgeschwindigkeit deutlich an ; jedoch kann hinsichtlich der Wirtschaftlichkeit des Verfahrens der Vorteil der höheren Reaktionsgeschwindigkeit durch den bei Anwendung von höherem Druck erforderlichen höheren apparativen Aufwand überkompensiert werden. Bevorzugt ist ein Druckbereich von Atmosphärendruck bis 10 bar (absolut), wobei das Arbeiten bei Atmosphärendruck besonders einfach auszuführen ist.

Das erfindungsgemäße Verfahren wird in der Regel bei einer Temperatur von 30°C bis zum Siedepunkt des wäßrigen Mediums, vorzugsweise von 50 bis 95°C, insbesondere 60 bis 90°C, durchgeführt.

Die Reaktion kann bezüglich des pH-Wertes unterschiedlich durchgeführt werden, wobei die pH-Führung einen Einfluß auf die Anteile der einzelnen Produkte im Endprodukt haben kann. So kann die Reaktion beispielsweise im durch die Reaktionsprodukte sauer werdenden Milieu, d.h. in einem pH-Bereich von unter pH 7 durchgeführt werden. Ebenso ist es möglich, während der Oxidation den pH-Wert durch Zugabe von Basen, Säuren oder Puffergemischen zu steuern, wobei in der Regel ein pH-Wert von weniger als 8 eingehalten wird. Aber auch bei höheren pH-Werten kann die Oxidation noch durchgeführt werden.

Beispielsweise ist es möglich, durch kontinuierliche Zugabe einer Base wie Natriumhydroxyd, Kaliumhydroxyd oder entsprechender wäßriger Lösungen dieser Basen den pH-Wert weitgehend konstant im Bereich von 6,5 bis 8, vorzugsweise 7 bis 7,5 einzustellen. Bei letzterer Verfahrensweise wird das Oxidationsprodukt Furan-2,5-dicarbonsäure in Form des Disalzes bevorzugt gebildet. Nach einer anderen Ausführungsform beginnt man die Oxidation bei etwa pH 7 und führt sie ohne Zusatz von Säuren oder Basen fort. Im sauren Milieu bilden sich bevorzugt 2,5-Diformylfuran und 5- Formylfuran-2-carbonsäure.

Das erfindungsgemäße Verfahren verläuft in einem Dreiphasensystem aus festem Katalysator, wäßrigem Medium und gasförmigem Sauerstoff. Es kann in allen Apparaturen, die sich für die Durchführung von Reaktionen in der Flüssigphase mit oder ohne Anwendung von Überdruck eignen, durchgeführt werden. Beispiele dafür sind die Durchführung in einem Rührkessel oder in einer Blasensäule mit suspendiertem Katalysator. Die Oxidation kann aber auch als Festbettreaktion mit gekörntem Katalysator in einem Rieselphasenreaktor durchgeführt werden.

Die für die Bildung des jeweils gewünschten Reaktionsproduktes erforderliche Reaktionszeit wird zweckmäßig dadurch bestimmt, daß man in gewissen Zeitabständen Proben der Reaktionslösung entnimmt und analysiert. Beispielsweise kann die Ausbeute der Reaktionsprodukte auf einfache Weise durch Analyse einer Probe mit Hilfe der Hochdruckflüssigkeits-Chromatographie im Vergleich zu Standardlösungen laufend bestimmt werden. Die Optimierung der Reaktionszeit ist zu empfehlen, da eine unnötig verlängerte Einleitung von Sauerstoff verstärkt zu Überoxidationen, in der Folge beispielsweise zu Decarboxylierungen, und damit zum Verlust an Ausbeute bei den gewünschten Reaktionsprodukten führen kann.

Das Reaktionsgemisch kann nach bekannten Methoden aufgearbeitet werden. In einem geeigneten Verfahren werden zunächst der Lösungsvermittler und das Wasser destillativ entfernt und eine anschließende Reinigung durch Kristallisation oder Extraktion vorgenommen.

Das erfindungsgemäße Verfahren besitzt gegenüber den anfangs genannten herkömmlichen Oxidationsverfahren den Vorteil, daß die Bildung unerwünschter Produkte, wie nitrose Gase oder Schwefelverbindungen, vermieden wird und auch die Abtrennung von überschüssigem Oxidationsmittel entfällt. Bei der erfindungsgemäßen katalytischen Oxidation entsteht neben den gewünschten Produkten zwangsweise nur noch Wasser, das ohnehin als Lösungsmittel verwendet wird.

Die Oxidationsprodukte des 5-Hydroxymethylfurfurals sind wertvolle Zwischenprodukte für die Herstellung von Kunststoffen, Tensiden und Harzen. Beispielsweise können die Furan-2,5-dicarbonsäure als Polyesterkomponente und die Aldehyde 2,5-Diformylfuran und 5-Formylfuran-2-carbonsäure nach Umsetzung mit langkettigen Aminen als Tenside oder in Polymerisations- und Copolymerisationsreaktionen zur Herstellung neuer Kunststoffe und Harze eingesetzt werden.

**Beispiele**

1) In ein von außen beheiztes senkrecht angeordnetes Glasrohr (Durchmesser : 50 mm, Länge : 1200 mm), das mit einer Mischung aus 162 g 5-Hydroxymethylfurfural, 1460 g Wasser und 81 g eines handelsüblichen Katalysators (5 Gew.-% Platin auf Aktivkohle) gefüllt ist, leitet man von unten durch eine Glasfritte 80 Normal-Liter pro Stunde Sauerstoff bei einer Temperatur von 70°C ein. Durch kontinuierliche Zugabe von 30%iger wäßriger Natronlauge wird der pH-Wert bei 7 bis 7,5 gehalten. Nach einer Reaktionszeit von 2,5 Stunden enthält die Reaktionslösung 234 g Furan-2,5-dicarbonsäure in Form des Dinatriumsalzes, entsprechend einer Ausbeute von 91% der Theorie.

2) In der im Beispiel 1 beschriebenen Apparatur werden 1500 g einer 20%igen wäßrigen Lösung von 5-Hydroxymethylfurfural in Gegenwart von 50 g des in Beispiel 1 verwendeten Katalysators bei einer Temperatur von 85°C mit 80 Normal-Liter pro Stunde Sauerstoff oxidiert. Nach einer Reaktionszeit von 11 Stunden, während der der pH-Wert durch Zugabe von 30%iger wäßriger Natronlauge bei 7 bis 7,5 gehalten wurde, enthält das Reaktionsgemisch 376 g Furan-2,5-dicarbonsäure in Form des Dinatriumsalzes, entsprechend einer Ausbeute von 79% der Theorie.

3) In der in Beispiel 1 beschriebenen Apparatur wird ein Gemisch aus 180 g 5-Hydroxymethylfurfural, 700 g Wasser, 700 g Diäthylenglykoldimethyläther und 75 g eines handelsüblichen Katalysators (5 Gew.-% Platin auf Aktivkohle) bei einer Temperatur von 60°C mit Sauerstoff umgesetzt. Im Gegensatz zu den Beispielen 1 und 2 wird keine Natronlauge zugesetzt, so daß der pH-Wert durch Bildung von Carboxylgruppen von anfangs etwa 7 auf unter 7 sinkt. Nach einer Reaktionszeit von 8 Stunden enthält das Reaktionsgemisch 122 g (61% der Theorie) 5-Formylfuran-2-carbonsäure, 43 g (24% der Theorie) 2,5-Diformylfuran und 18 g (8% der Theorie) Furan-2,5-dicarbonsäure.

4) Die in Beispiel 1 beschriebene Umsetzung wird 4 Stunden bei 60°C unter sonst gleichen Bedingungen durchgeführt. Die Reaktionslösung enthält 252 g Furan-2,5-dicarbonsäure in Form des Dinatriumsalzes, entsprechend einer Ausbeute von 98% der Theorie.

**Patentansprüche**

1. Verfahren zur Oxidation mindestens der Hydroxymethylgruppe von 5-Hydroxymethylfurfural, wobei 5-Hydroxymethylfurfural in wäßrigem Medium mit Sauerstoff als Oxidationsmittel in Gegenwart eines Katalysators, der mindestens ein Platinmetall enthält, oxidiert wird, dadurch gekennzeichnet, dass die Oxidation bei einem pH-Wert von höchstens 8 durchgeführt wird.

2. Verfahren nach Anspruch 1, dadurch gekennzeichnet, daß der Katalysator als Platinmetall Palladium, Platin oder ein Gemisch von Palladium und Platin, insbesondere nur Platin, enthält.

3. Verfahren nach Anspruch 1 oder 2, dadurch gekennzeichnet, daß der Katalysator aus 1 bis 10 Gew.-% Platinmetall und einem Träger, vorzugsweise Aktivkohle besteht.

4. Verfahren nach einem oder mehreren der Ansprüche 1 bis 3, dadurch gekennzeichnet, daß die Oxidation in einem Druckbereich von 0,5 bis 100 bar, vorzugsweise von Atmosphärendruck bis 10 bar und insbesondere bei Atmosphärendruck, durchgeführt wird.

5. Verfahren nach einem oder mehreren der Ansprüche 1 bis 4, dadurch gekennzeichnet, daß das wäßrige Medium einen unter den Reaktionsbedingungen gegenüber den Reaktionsteilnehmern inerten Lösungsvermittler, vorzugsweise in einer Menge von 10 bis 75 Gew.-%, insbesondere 30 bis 50 Gew.-%, enthält, bezogen auf die Menge an Wasser und Lösungsvermittler.

6. Verfahren nach Anspruch 5, dadurch gekennzeichnet, daß der Lösungsvermittler ein Glykoläther ohne Hydroxygruppen ist, vorzugsweise der Formel $R^1O[CH_2CH(CH_3)O]_nR^2$, wobei n eine ganze Zahl von 1 bis 4 ist und $R^1$ und $R^2$ jeweils unabhängig voneinander $C_1$-$C_4$-Alkyl bedeuten, insbesondere Diäthylenglykoldimethyläther ist.

7. Verfahren nach einem oder mehreren der Ansprüche 1 bis 6, dadurch gekennzeichnet, daß die Oxidation bei einer Temperatur von 30°C bis zum Siedepunkt des wäßrigen Mediums, vorzugsweise von 50 bis 95°C, insbesondere von 60 bis 90°C, durchgeführt wird.

8. Verfahren nach einem oder mehreren der Ansprüche 1 bis 7, dadurch gekennzeichnet, daß das wäßrige Medium am Beginn der Oxidation 5 bis 30 Gew.-% bezogen auf die Menge von Wasser und Lösungsvermittler, 5-Hydroxymethylfurfural enthält.

9. Verfahren nach einem oder mehreren der Ansprüche 1 bis 8, dadurch gekennzeichnet, daß der pH-Wert

bei der Oxidation durch Zusatz einer Base im pH-Bereich von 6,5 bis 8, vorzugsweise 7 bis 7,5 gehalten wird.

10. Verfahren nach einem oder mehreren der Ansprüche 1 bis 9, dadurch gekennzeichnet, daß die Oxidation bei etwa pH 7 beginnt und ohne Zusatz von Säuren oder Basen durchgeführt wird.

## Claims

1. A process for the oxidation of at least the hydroxymethyl group of 5-hydroxymethylfurfural, in which 5-hydroxymethylfurfural is oxidized in an aqueous medium using oxygen as the oxidizing agent in the presence of a catalyst which contains at least one metal from the platinum group, which process comprises carrying out the oxidation at a pH of at most 8.

2. The process as claimed in claim 1, wherein the catalyst comprises palladium, platinum or a mixture of palladium and platinum, in particular only platinum, as the platinum metal.

3. The process as claimed in claim 1 or 2, wherein the catalyst comprises 1 to 10% by weight of platinum metal and a support, preferably activated carbon.

4. The process as claimed in one or more of claims 1 to 3, wherein the oxidation is carried out in a pressure range from 0.5 to 100 bar, preferably atmospheric pressure to 10 bar and in particular at atmospheric pressure.

5. The process as claimed in one or more of claims 1 to 4, wherein the aqueous medium contains a solubilizing agent which is inert toward the reactants under the reaction conditions, preferably in an amount of 10 to 75% by weight, in particular 30 to 50% by weight, relative to the amount of water and solubilizing agent.

6. The process as claimed in claim 5, wherein the solubilizing agent is a glycol ether without hydroxyl groups, preferably of the formula $R^1O[CH_2CH(CH_3)O]_nR^2$, in which n is an integer from 1 to 4 and $R^1$ and $R^2$, each independently of one another, are $C_1$-$C_4$-alkyl, in particular diethylene glycol dimethyl ether.

7. The process as claimed in one or more of claims 1 to 6, wherein the oxidation is carried out at a temperature from 30°C to the boiling point of the aqueous medium, preferably 50 to 95°C, in particular 60 to 90°C.

8. The process as claimed in one or more of claims 1 to 7, wherein the aqueous medium contains 5 to 30% by weight, relative to the amount of water and solubilizing agent, of 5-hydroxymethylfurfural at the beginning of the oxidation.

9. The process as claimed in one or more of claims 1 to 8, wherein the pH during the oxidation is maintained in the pH range from 6.5 to 8, preferably 7 to 7.5, by adding a base.

10. The process as claimed in one or more of claims 1 to 9, wherein the oxidation begins at about a pH of 7 and is carried out without adding acids or bases.

## Revendications

1. Procédé pour oxyder au moins le radical hydroxyméthyl de l'hydroxyméthyl-5 furfural, par oxydation de l'hydroxyméthyl-5 furfural en milieu aqueux, au moyen d'oxygène comme agent d'oxydation, en présence d'un catalyseur renfermant au moins un platinoïde, procédé caractérisé en ce qu'on effectue l'oxydation à un pH d'au plus 8.

2. Procédé selon la revendication 1 caractérisé en ce que le catalyseur contient, comme platinoïde, du palladium, du platine ou un mélange de palladium et de platine, en particulier du platine seulement.

3. Procédé selon l'une des revendications 1 et 2, caractérisé en ce que le catalyseur est constitué de 1 à 10% en poids d'un platinoïde et d'un support, qui est de préférence du charbon actif.

4. Procédé selon l'une quelconque des revendications 1 à 3, caractérisé en ce qu'on effectue l'oxydation dans un intervalle de pression allant de 0,5 à 100 bar, de préférence de la pression atmosphérique à 10 bar ou, mieux encore, sous la pression atmosphérique.

5. Procédé selon l'une quelconque des revendications 1 à 4, caractérisé en ce que le milieu aqueux contient un adjuvant de dissolution inerte à l'égard des partenaires réactionnels dans les conditions de la réaction, de préférence en une quantité de 10 à 75% en poids, plus particulièrement de 30 à 50% en poids, par rapport à la quantité d'eau et d'adjuvant de dissolution.

6. Procédé selon la revendication 5 caractérisé en ce que l'adjuvant de dissolution est un éther de glycol sans radicaux hydroxy, qui répond de préférence à la formule $R^1O[CH_2CH(CH_3)O]_nR^2$ dans laquelle n désigne un nombre entier de 1 à 4 et $R^1$ et $R^2$ représentent chacun, indépendamment l'un de l'autre, un alkyle en $C_1$-$C_4$, plus particulièrement l'éther diméthylique du diéthylène-glycol.

7. Procédé selon l'une quelconque des revendications 1 à 6, caractérisé en ce que l'oxydation est effectuée à une température comprise entre 30°C et le point d'ébullition du milieu aqueux, de préférence entre 50 et 95°C, plus particulièrement entre 60 et 90°C.

8. Procédé selon l'une quelconque des revendications 1 à 7, caractérisé en ce que le milieu aqueux contient, au début de l'oxydation, de 5 à 30% en poids d'hydroxyméthyl-5 furfural par rapport à la quantité d'eau et d'adjuvant de dissolution.

9. Procédé selon l'une quelconque des revendications 1 à 8, caractérisé en ce que le pH est maintenu, au cours de l'oxydation, dans l'intervalle allant de 6,5 à 8, de préférence de 7 à 7,5, par addition d'une base.

10. Procédé selon l'une quelconque des revendications 1 à 9, caractérisé en ce que l'oxydation commence à un pH d'environ 7 et est effectuée sans addition d'acides ni de bases.